(19) Europäisches Patentamt
European Patent Office
Office européen des brevets

(11) Veröffentlichungsnummer: **0 482 024 B1**

(12) **EUROPÄISCHE PATENTSCHRIFT**

(45) Veröffentlichungstag der Patentschrift: **24.05.95**

(51) Int. Cl.$^{6}$: **C09K 19/34**

(21) Anmeldenummer: **90909694.3**

(22) Anmeldetag: **27.06.90**

(86) Internationale Anmeldenummer:
**PCT/EP90/01020**

(87) Internationale Veröffentlichungsnummer:
**WO 91/00897 (24.01.91 91/03)**

# (54) CHIRALE ODER ACHIRALE RINGVERBINDUNGEN.

(30) Priorität: **11.07.89 DE 3922790**
**03.03.90 DE 4006743**

(43) Veröffentlichungstag der Anmeldung:
**29.04.92 Patentblatt 92/18**

(45) Bekanntmachung des Hinweises auf die Patenterteilung:
**24.05.95 Patentblatt 95/21**

(84) Benannte Vertragsstaaten:
**DE GB**

(56) Entgegenhaltungen:
**EP-A- 0 255 236**
**EP-A- 0 360 521**
**EP-A- 0 392 432**
**WO-A-88/05803**
**WO-A-88/08441**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestrasse 34**
**W-6103 Griesheim (DE)**
Erfinder: **POETSCH, Eike**
**Am Buchwald 4**
**D-6109 Mühltal 6 (DE)**
Erfinder: **GEELHAAR, Thomas**
**Trajanstrasse 12**
**W-6500 Mainz (DE)**
Erfinder: **HITTICH, Reinhard**
**Am Kirchberg 11**
**D-6101 Modautal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 482 024 B1

## Beschreibung

Die Erfindung betrifft chirale oder achirale Ringverbindungen der Formel I

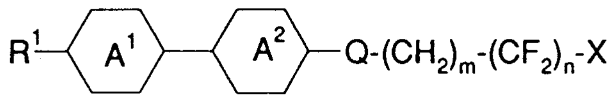

I

worin

- $R^1$ eine geradkettige Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH = CH-Gruppen und/oder-CHHalogen- und/oder -CHCN-Gruppen und/oder -O-CO-CHHalogen- und/oder -CO-O-CHCN-Gruppen ersetzt sein können, oder $X-(CF_2)_n-(CH_2)_m-Q-$,
- X H oder F,
- $A^1$ und $A^2$ jeweils unabhängig voneinander unsubstituiertes oder durch ein oder zwei F-Atome substituiertes 1,4-Phenylen, worin auch eine oder zwei CH-Gruppen durch N ersetzt sein können,
- Q -O-,
- m 1 bis 10, und
- n 2 bis 8 bedeutet.

Die Verbindungen der Formel I können wie ähnliche in DE-OS 35 15 373 und DE-OS 35 15 374 beschriebene Verbindungen als Komponenten chiraler getilteter smektischer flüssigkristalliner Phasen verwendet werden.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44, (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 $\mu$m) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ähnliche Verbindungen mit Perfluoralkylgruppen als Flügelgruppen, welche als Komponenten ferroelektrischer Medien eingesetzt werden können, sind bereits bekannt.

Aus der japanischen Offenlegungsschrift JP-63-27451 sind z.B. optisch aktive flüssigkristalline Verbindungen bekannt, welche neben einer Perfluoralkylgruppe ein chirales Kohlenstoffatom aufweisen, wobei die chirale Gruppe sich von (-)-2-Methylbutanol ableitet. Diese Verbindungen weisen kleine Spontanpolarisationen auf und dürften daher aufgrund ihrer Schaltzeiten kaum technisch anwendbar sein.

Aus der EP 0 255 236 sind ebenfalls chirale Polyfluoralkylverbindungen mit zwei Ringen bekannt, die jedoch keine smektische C-Phasen aufweisen.

Aus der japanischen Offenlegungsschrift JP 1-104031 sind z.B. achirale und chirale flüssigkristalline Verbindungen bekannt, welche eine Perfluoralkyl- oder Polyfluoralkylgruppe aufweisen.

In der WO 88/05803 und der WO 88/08441 werden ähnliche Polyfluoralkylverbindungen beschrieben.

Bei diesen Verbindungen ist die Perfluoralkylgtuppe entweder über eine Carboxygruppe oder über eine Methylen- bzw. Ethylencarboxyloxygruppe mit dem mesogenen Rest verknüpft. Die dort beschriebenen Verbindungen weisen in der Regel keine oder nur sehr schmale $S_c$-Phasen auf.

Die EP 0 392 432 stellt einen Stand der Technik gemäß Artikel 54(3) EPÜ dar und offenbart ferroelektrische Mischungen mit einem sehr geringen Gehalt an Polyfluoralkylverbindungen (0,01 bis 0,1 Gew.%),
sowie eine Verbindung der Formel

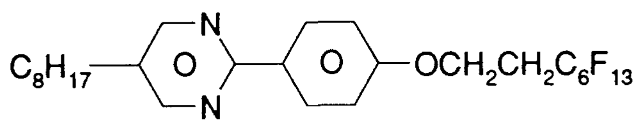

welche durch die Maßgabe ausgenommen ist.

Ein großer Nachteil für viele Anwendungen der derzeit verfügbaren Materialien mit chiralen getilteten smektischen Phasen (wie z.B. Sc*) ist deren relativ hohe optische Anisotropie, die durch relativ hohe Viskositätswerte bedingten nicht ausreichend kurzen Schaltzeiten, sowie, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3).

Es wurde nun gefunden, daß die Verwendung von Verbindungen der Formel I als Komponenten chiraler getilteter smektischer Mischungen die erwähnten Nachteile wesentlich vermindern kann. Die chiralen Verbindungen der Formel I besitzen insbesondere einen vergleichsweise großen Pitch, womit eine problemlose Orientierung in der Flüssigkristallzelle ermöglicht wird, und sind somit als Komponenten chiraler getilteter smektischer flüssigkristalliner Phasen vorzüglich geeignet. Insbesondere sind mit ihrer Hilfe chemisch besonders stabile chirale getiltete smektische flüssigkristalline Phasen mit günstigen ferroelektrischen Phasenbereichen, insbesondere mit breiten Sc*-Phasenbereichen, negativer oder auch positiver dielektrischer Anisotropie, niedriger optischer Anisotropie, günstiger Pitchhöhe, niedriger Viskosität und für derartige Phasen hohen Werten für die spontane Polarisation und sehr kurzen Schaltzeiten herstellbar. P ist die spontane Polarisation in $nC/cm^2$. Die achiralen Verbindungen der Formel I weisen insbesondere hohe $S_C/S_A$-Übergänge, niedrige optische Anisotropie und günstige Viskositäten auf.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation und/oder den Phasenbereich und/oder den Tiltwinkel und/oder den Pitch und/oder die Schaltzeiten einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und weisen günstige Werte der optischen Anisotropie auf. Teilweise zeigen die Verbindungen der Formel I flüssigkristalline Mesophasen in einem für die elektrooptische Verwendung günstig gelegenen Temperaturbereich, es können jedoch auch isotrope oder monotrop flüssigkristalline Verbindungen der Formel I als Komponenten chiraler getilteter smektischer Phasen vorteilhaft eingesetzt werden. Chemisch, thermisch und gegen Licht sind sie sehr stabil.

Gegenstand der Erfindung sind somit chirale oder achirale Ringverbindungen der Formel I

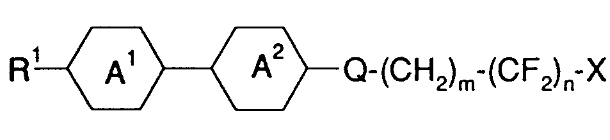

I

worin

$R^1$ eine geradkettige Alkyl- oder Perfluoralkyl-Gruppe mit jeweils 1-12 C-Atomen, worin auch

eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder-CHHalogen- und/oder -CHCN-Gruppen und/oder -O-CO-CHHalogen- und/oder -CO-O-CHCN-Gruppen ersetzt sein können, oder $X-(CF_2)_n-(CH_2)_m-Q$,

X H oder F,

$A^1$ und $A^2$ jeweils unabhängig voneinander unsubstituiertes oder durch ein oder zwei F-Atome substituiertes 1,4-Phenylen, worin auch eine oder zwei CH-Gruppen durch N ersetzt sein können,

Q -O-,

m 1 bis 10, und

n 2 bis 8 bedeutet.

mit der Maßgabe, daß die Verbindung der Formel

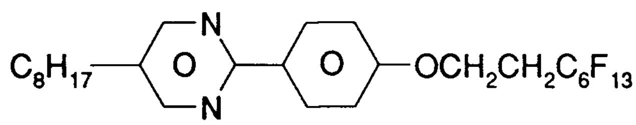

ausgenommen ist.

Ein weiterer Gegenstand der Erfindung sind chirale getiltete smektische flüssigkristalline Phase mit mindestens zwei flüssigkristallinen Komponenten, dadurch gekennzeichnet, daß sie mindestens eine Verbindung der Formel I nach Anspruch 1 oder 2 enthält, sowie chirale getiltete smektische flüssigkristalline Phase mit mindestens zwei flüssigkristallinen Komponenten, dadurch gekennzeichnet, daß sie mindestens eine Verbindung der Formel I

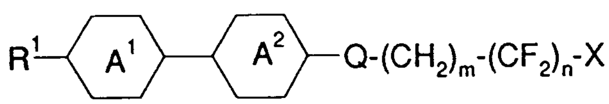

I

worin

$R^1$ eine geradkettige Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH=CH-Gruppen und/oder-CHHalogen- und/oder -CHCN-Gruppen und/oder -O-CO-CHHalogen- und/oder -CO-O-CHCN-Gruppen ersetzt sein können, oder $X-(CF_2)_n-(CH_2)_m-Q-$,

X H oder F,

$A^1$ und $A^2$ jeweils unabhängig voneinander unsubstituiertes oder durch ein oder zwei F-Atome substituiertes 1,4-Phenylen, worin auch eine oder zwei CH-Gruppen durch N ersetzt sein können,

Q -O-,

m 1 bis 10, und

n 2 bis 8 bedeutet, enthält, mit der Maßgabe, daß Phasen mit 0,01 bis 1 Gew.-% einer Verbindung der Formel I ausgenommen sind.

Weiterhin Gegenstand der Erfindung ist die Verwendung der Verbindungen der Formel I nach Anspruch 1 oder 2 als Komponenten flüssigkristalliner Phasen, sowie ein elektrooptische Anzeigeelement, welches als Dielektrikum eine Phase nach einem der Ansprüche 3 bis 5 enthält.

Vor- und nachstehend haben $R^1$, $A^1$, $A^2$, Q, X, m und n die angegebene Bedeutung, sofern nicht ausdrücklich etwas anderes vermerkt ist.

Der Rest $-(CH_2)_m-(CF_2)_n-X$ wird im folgenden als $R_{F,H}$ bezeichnet.

Die Verbindungen der Formel I umfassen dementsprechend insbesondere die bevorzugten Verbindungen der Teilformeln Ia bis If:

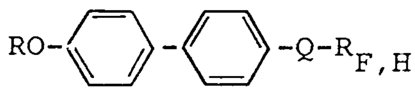

Ia

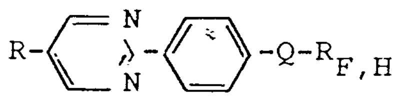

Ib

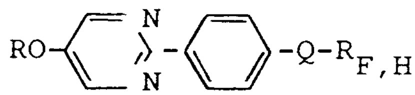

Ic

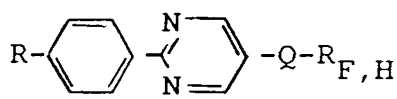

Id

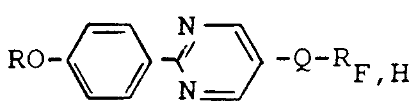

Ie

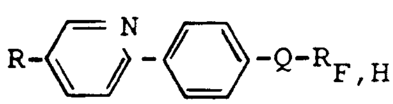

If

Darunter sind diejenigen der Formeln Ib, Ic und Ie besonders bevorzugt.

In den bevorzugten Verbindungen der vor- und nachstehenden Formeln können die Alkylreste, in denen auch eine $CH_2$-Gruppe (Alkoxy bzw. Oxaalkyl) durch ein 0-Atom ersetzt sein kann, geradkettig oder verzweigt sein. Vorzugsweise haben sie 5, 6, 7, 8, 9 oder 10 C-Atome und bedeuten demnach bevorzugt Pentyl, Hexyl, Heptyl, Octyl, Nonyl, Decyl, Pentoxy, Hexoxy, Heptoxy, Octoxy, Nonoxy oder Decoxy, ferner auch Ethyl, Propyl, Butyl, Undecyl, Dodecyl, Propoxy, Ethoxy, Butoxy, Undecoxy, Dodecoxy, 2-Oxapropyl (= 2-Methoxymethyl), 2- (= Ethoxymethyl) oder 3-Oxabutyl (= 2-Methoxypentyl), 2-, 3- oder 4-Oxapentyl, 2-, 3-, 4- oder 5-Oxahexyl, 2-, 3-, 4-, 5- oder 6-Oxaheptyl.

$A^1$ und $A^2$ sind bevorzugt jeweils unabhängig voneinander eine 1,4-Phenylen- (Phe), eine Pyrimidin-2,5-diyl-(Pyr), eine Pyridin-2,5-diyl- (Pyn), eine Pyrazin-3,6-diyl- oder eine Pyridazin-2,5-diyl-Gruppe, insbesondere bevorzugt Phe, Pyr oder Pyn. Vorzugsweise enthalten die erfindungsgemäßen Verbindungen nicht mehr als eine 1,4-Phenylengruppe, worin eine oder zwei CH-Gruppen durch N ersetzt sind.

Besonders bevorzugt sind Verbindungen der Formel I und der vorstehenden Teilformeln, die eine Gruppierung -Phe-Phe-, Phe-Pyr oder Phe-Pyn enthalten. Besonders bevorzugt sind die Gruppen

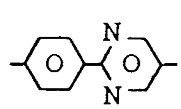 und 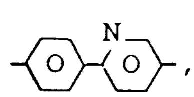,

wobei die 1,4-Phenylenringe auch durch ein oder zwei Fluor-Atome substituiert sein können, sowie ferner unsubstituiertes oder ein- oder mehrfach durch Fluor substituiertes 4,4'-Biphenylyl.

Der Rest $R^1$ kann auch ein optisch aktiver organischer Rest mit einem asymmetrischen Kohlenstoffatom sein.

Unter den Verbindungen der Formel I sowie Ia bis If sind diejenigen bevorzugt, in denen mindestens einer der darin enthaltenen Reste eine der angegebenen bevorzugten Bedeutungen hat.

Q ist -O-.

$R^1$ ist vorzugsweise geradkettiges Alkyl oder Alkoxy mit 5 bis 12 C-Atomen.

m ist 1 bis 10, vorzugsweise 3 bis 6.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können zur Herstellung von Verbindungen der Formel I geeignete Vorstufen der Formel $X-(CF_2)_n-(CH_2)_m-Y$ oder $X-(CF_{2n}-(CH_2))_{m-1}-Y$ (Y = Halogen, z.B. J, -OH, OTs oder OTf (Tosylat oder Triflat)) eingesetzt werden, die bekannt sind oder in Analogie zu bekannten Verbindungen hergestellt werden können.

Wie von F. Tournilhac und J. Simon beschrieben (FLC 89, 27.-30 Juni 1989, Göteborg, Schweden, Poster 82), kann man nach literaturbekannten Methoden (z.B., nach J.D. Park et al., J. Org. Chem. 26, 2089 (1961) oder N.O. Brace J. Org. Chem. 27, 3033 (1962)) Perfluoralkyljodide radikalisch, beispielsweise mit Azobisisobutyronitril (AIBN) als Kettenstarter, an ω-Hydroxy-1-alkene bzw. an deren Acetate addieren und anschließend die entstandenen Jodide entweder durch katalytische Hydrierung oder mit Zink und Säure zu den semifluorierten Alkoholen bzw. deren Acetaten reduzieren. Die Alkohole, die man entweder direkt oder durch die Verseifung der Acetate erhält, werden in die Tosylate und anschließend nach Finkelstein in die entsprechenden primären Jodide überführt (siehe Reaktionsschema).

$$C_nF_{2n+1}J + CH_2{=}CH-(CH_2)_{m-2}-X \xrightarrow{\text{AIBN}} C_nF_{2n+1}CH_2-CHJ(CH_2)_{m-2}-X$$

$$\xrightarrow[\text{Zn/H+ oder Ni/H}_2]{\text{Reduktion}} C_nF_{2n+1}(CH_2)_m-X \longrightarrow C_nF_{2n+1}(CH_2)_m-OTs$$

$$\xrightarrow{\text{Finkelstein}} C_nF_{2n+1}(CH_2)_m-J$$

X = OH oder OAc

Die Tosylate eignen sich bevorzugt zur Alkylierung phenolischer Hydroxygruppen.

Verbindungen der Formel I, erhält man nach üblichen Veretherungsverfahren, zum Beispiel durch Umsetzung der Verbindungen der Formel $X-(CF_2)_n-(CH_2)_m-OTf$ mit entsprechenden Phenolaten.

Weitere Synthesemöglichkeiten für weitere bevorzugte Verbindungen sind in den folgenden Schemata angegeben:

Schema 1

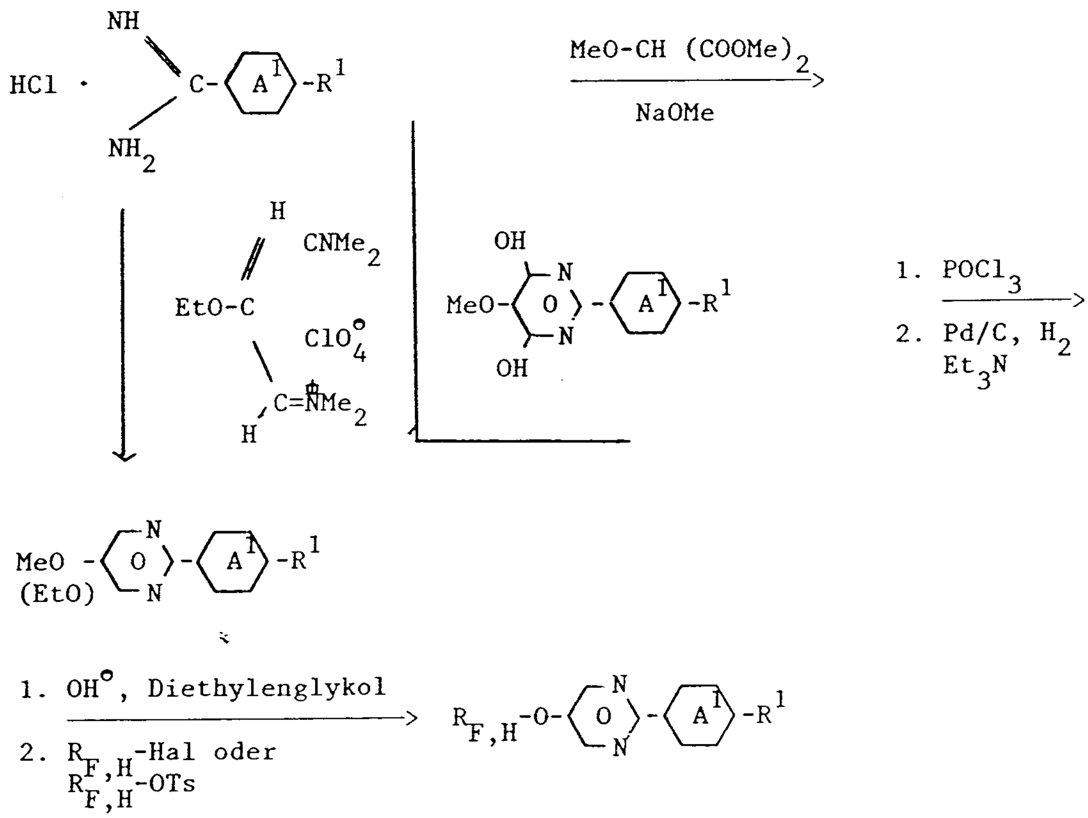
HCl ·
MeO-CH (COOMe)2
NaOMe
ClO4
1. POCl3
2. Pd/C, H2
Et3N
1. OH, Diethylenglykol
2. RF,H-Hal oder
RF,H-OTs

Schema 2

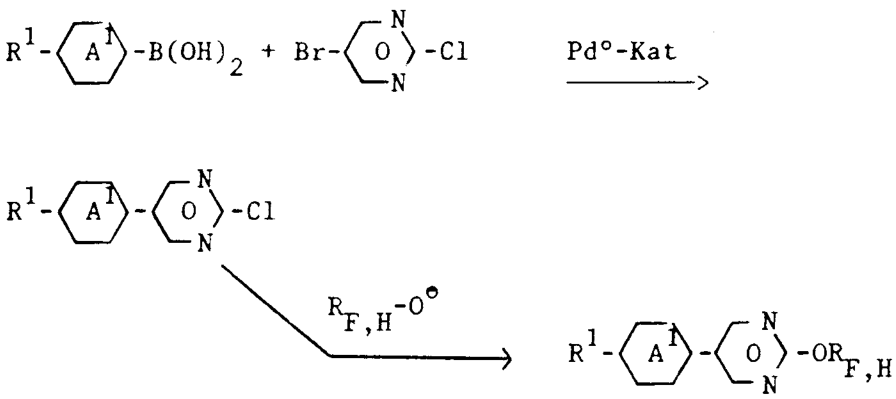


Schema 3

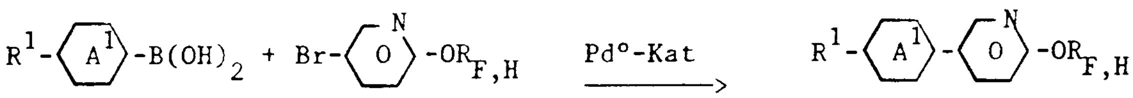


Schema 4

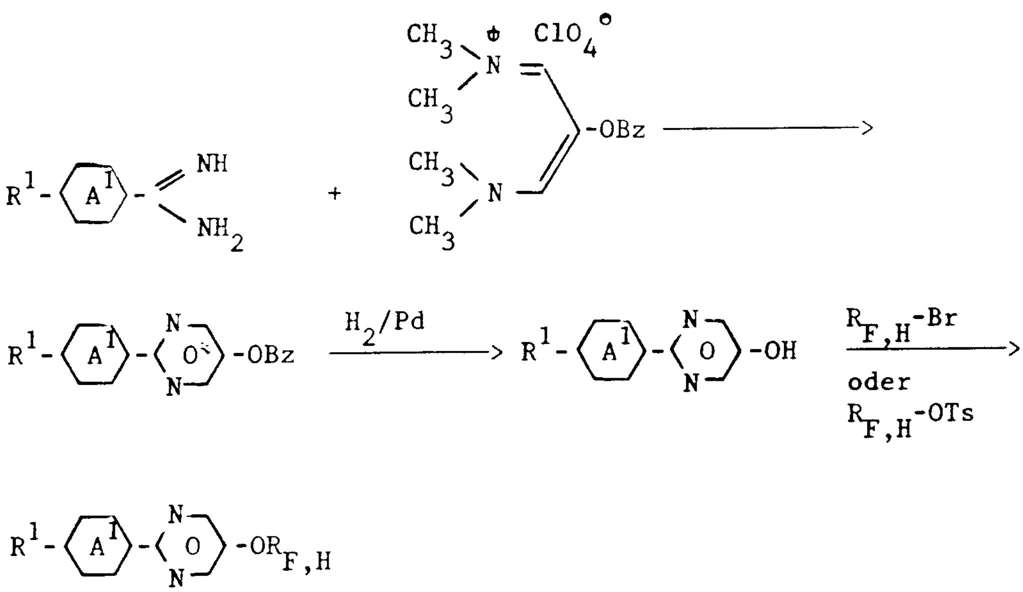

Schema 5

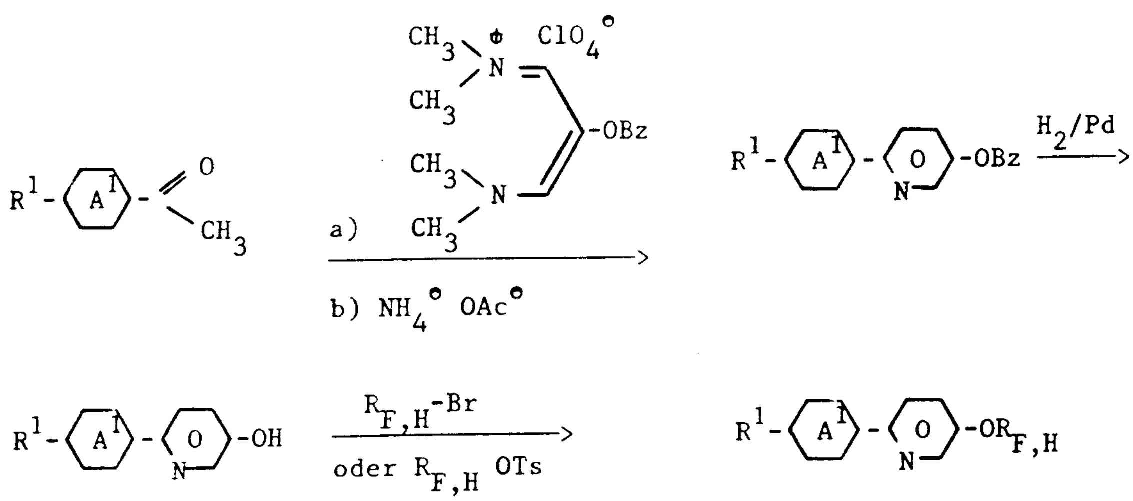


Schema 6

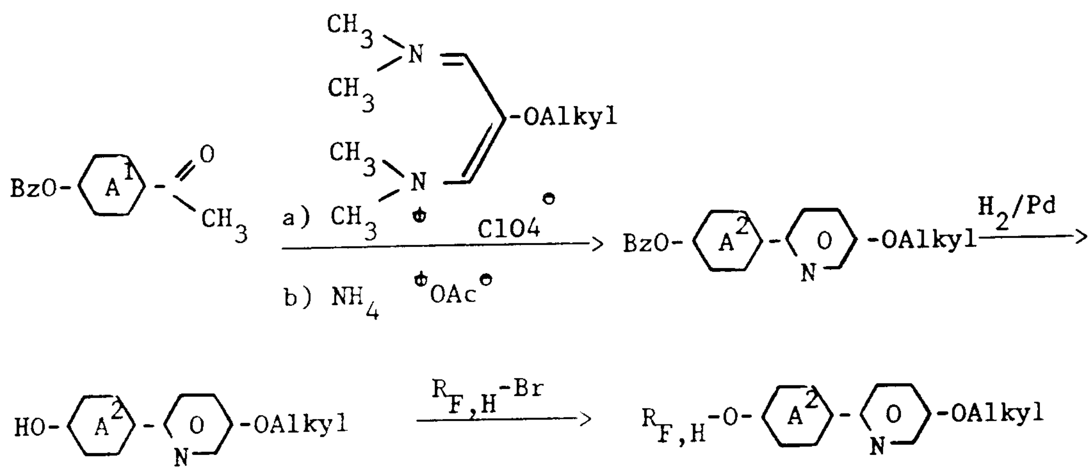


Die Einführung der heterocyclischen Strukturelemente kann einerseits dadurch erfolgen, daß man Vorstufen, die diese Strukturelemente bereits enthalten, nach den bekannten Methoden zu den Verbindungen der Formel I umsetzt. Andererseits können aber auch in entsprechend strukturierten Vorstufen oder Unterstruktureinheiten der Verbindungen der Formel I nach an sich bekannten Methoden Heterocyclenreste erzeugt werden.

Die 2,5-disubstituierten Pyrimidine können beispielsweise durch Umsetzung entsprechender Amidinhydrochloride (herstellbar aus den entsprechenden Carbonsäuren) mit Malondialdehydtetramethylacetalen nach an sich bekannten Methoden hergestellt werden. Die 2,5-disübstituierten Pyrimidine sind durch Kopplung von metallorganischen Zinkverbindungen mit entsprechenden Brompyridinderivaten entsprechend DE-OS 36 32 410 erhältlich. Die 2,5-disubstituierten Pyrazine sind erhältlich durch Kondensation von geeignet substituierten Ethylendiaminen mit Glyoxalderivaten, Oxidation der Dihydroverbindungen mit Luftsauerstoff oder anderen Oxidationsmitteln und Isolierung der gewünschten 2,5-disubstituierten Pyrazine aus dem entstandenen Gemisch der 2,5- und 2,6-Disubstitutionsprodukte. Die 3,6-disübstituierten Pyridazine sind zugänglich durch Umsetzung von 1,4-Diketonen (hergestellt z.B. nach Stetter durch thiazoliumsalzkatalysierte Addition eines Aldehyds an ein $\alpha,\beta$-ungesättigtes Keton) und anschließende Oxidation des Dihydropyridazins mit Luftsauerstoff oder anderen Oxidationsmitteln wie Kaliumnitrit oder Chromsäure in Eisessig.

Im folgenden wird die Synthese einiger besonders interessanter Hydroxy-Zwischenstufen beschrieben:

a) 5-Alkyl-2-(2,3-difluor-4-hydroxyphenyl)-pyridine sind erhältlich durch Umsetzung von 2,3-Difluor-4-benzyloxy-benzamidinhydrochlorid mit 2-Alkyl-3-ethoxy-acroleinen bzw. mit in 2-Stellung alkylierten

Malonaldehydtetraacetalen oder entsprechend substituierten vinylogen Fornamidiniumsalzen (R.M. Wagner und Ch. Jutz, Chem. Ber. 104, 2975 (1971)), indem man vorzugsweise die Komponenten in DMF (Dimethylformamid) erhitzt und anschließend die Schutzgruppe abspaltet.

b) 5-Hydroxy-2-(2,3-difluor-4-alkylphenyl)-pyrimidine bzw. 5-Hydroxy-2(2,3-difluor-4-alkoxyphenyl)-pyrimidine sind erhältlich durch Kondensation von 4-Alkyl- bzw. 4-Alkoxy-2,3-difluorbenzamidinhydrochlorid mit 2-Benzyloxytrimethiniumperchlorat (A. Holy, Z. Arnold; Collection Czechoslov. Chem. Comm. 38, 1371-1380 (1973), oder 2-Benzyloxy-3-dimethylaminoacrolein (H. Horstmann et al., Arzneimittelforsch. 11, 682 (1961), und anschließender Hydrogenolyse der Benzylgruppe.

c) 5-Hydroxy-2(2,3-difluor-4-alkylphenyl)pyridine bzw. 5-Hydroxy-2(2,3-difluor-4-alkoxyphenyl)pyridine sind erhältlich aus 2-Benzyloxytrimethiniumsalz durch Kondensation mit 4-Alkyl- oder 4-Alkoxy-2,3-difluoracetophenonen, Umsetzung mit $NH_3/NH_4Cl$ oder Ammoniumacetat.

Analog den Vorschriften von Ch. Jutz et al. (Liebigs Ann. Chem. 1975, 874-900) und anschließende Hydrogenolyse oder aus 4-Alkyl- bzw. 4-Alkoxy-2,3-difluorphenylboronsäure durch Kopplung mit 5-Acetoxy-2-brompyridin (erhältlich aus 5-Hydroxy-2-brompyridin durch Veresterung) in Gegenwart eines Pd-Katalysators entsprechend den Arbeiten von Suzuki et al. (Synth. Commun. 11, 513-19 (1981).

d) 5-Alkoxy-2(2,3-difluor-4-hydroxyphenyl)pyridine sind erhältlich durch Kopplung von 2,3-Difluor-4-benzyloxyphenylboronsäure mit 5-Alkoxy-2-brompyridin entsprechend obengenannter Literatur und anschießender Hydrogenolyse.

e) 5-Alkyl-2(2,3-difluor-4-hydroxyphenyl)pyridine sind erhältlich durch Kopplung von 2-Brom-5-methylpyridin mit 2,3-Difluor-4-benzyloxyphenylboronsäure und einem Pd-Katalysator unter den bereits genannten Bedingungen, Kettenverlängerung der Methylgruppe durch Deprotonierung mit LDA als Base (-65 °C) und Alkylierung mit einem Alkylbromid und Hydrogenolyse.

f) 5-Alkyl-2(2,3-difluor-4-hydroxyphenyl)pyrimidine bzw. 5-Alkoxy-2(2,3-difluor-4-hydroxyphenyl)-pyrimidine sind herstellbar durch die übliche Kondensation von 2,3-Difluor-4-benzyloxybenzamidin mit 2-Alkylmalonaldehydtetraacetalen oder 2-Alkyl-3-ethoxyacroleinen bzw. 2,3-Dialkoxyacroleinen oder der entsprechenden Immoniumsalze oder Alkoxytrimethiniumsalzen und anschließender Hydrogenolyse.

Die optisch aktiven Verbindungen der Formel I erhält man durch den Einsatz entsprechender optisch aktiver Ausgangsmaterialien und/oder durch Trennung der optischen Antipoden mittels Chromatographie nach bekannten Methoden.

Die erfindungsgemäßen Phasen enthalten mindestens eine, vorzugsweise mindestens zwei Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Die Chiralität beruht vorzugsweise teilweise oder vollständig auf chiralen Verbindungen der Formel I. Diese Phasen enthalten vorzugseise eine oder zwei chirale Verbindungen der Formel I. Es können jedoch auch achirale Verbindungen der Formel I (zum Beispiel in Form eines Racemates) eingesetzt werden, wobei dann die Chiralität der Phase durch andere optisch aktive Verbindungen hervorgerufen wird. Falls chirale Verbindungen der Formel I zum Einsatz kommen, eignen sich neben den reinen optischen Antipoden auch Gemische mit einem Enantiomerenüberschuß. Die oben erwähnten weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp:

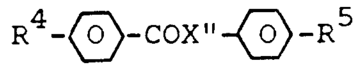 Va

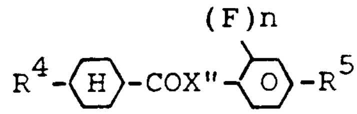 Vb

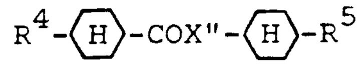 Vc

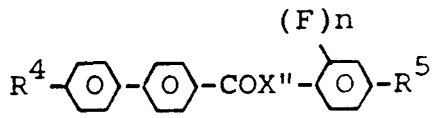 Vd

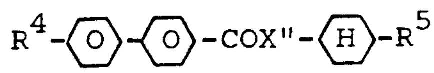 Ve

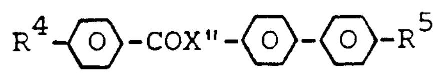 Vf

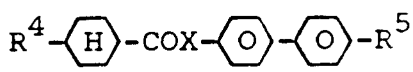 Vg

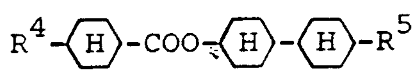 Vh

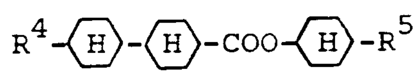 Vi

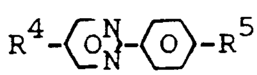 Vj

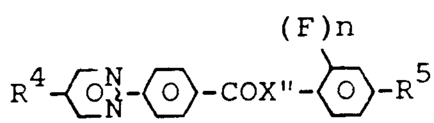 Vk

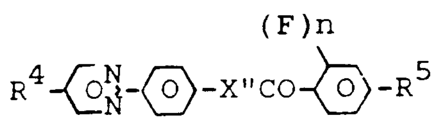 Vl

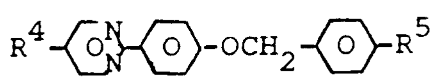 Vm

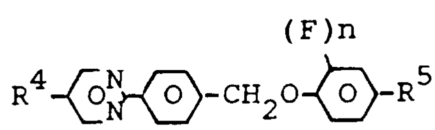 Vn

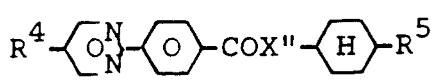 Vo

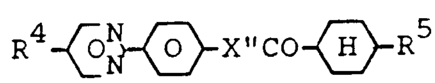 Vp

$R^4$ und $R^5$ sind jeweils Alkyl mit 1 bis 15 C-Atomen, worin auch eine $CH_2$-Gruppe durch -O-, -CO-O- oder -O-CO- ersetzt sein kann, vorzugsweise geradkettiges oder verzweigtes Alkyl, Alkoxy, Alkanoyloxy oder Alkoxycarbonyl mit jeweils 3 bis 12 C-Atomen. X" ist O oder S, vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

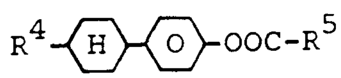

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement M, N oder O.

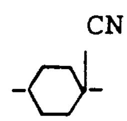

M

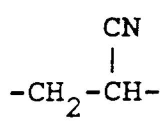

N

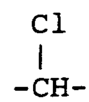

O

Bevorzugte Verbindungen dieser Art entsprechen den Formeln VIb und VIc:

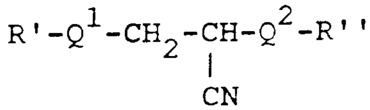

VIb

R'-$Q^3$-$Q^4$-R''' VIc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

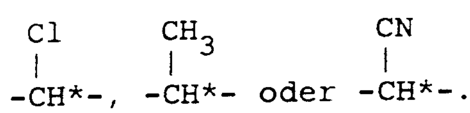

R''' hat vorzugsweise die Formel

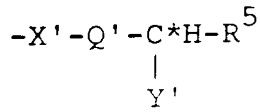

worin $R^5$ die angegebene Bedeutung besitzt, und

X' -O-, -CO-O- oder -O-CO-,

Q' $(CH_2)_p$, worin p 0, 1, 2 oder 3 bedeutet, und

Y' F, Cl, CN oder $CH_3$

bedeuten.

Besonders bevorzugte Komponenten mit negativer dielektrischer Anisotropie sind die in der WO 86-00529 beschriebenen Verbindungen mit dem Strukturelement M oder N. Besonders bevorzugt sind diejenigen der Formel VId

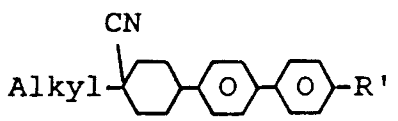

VId

worin Alkyl eine geradkettige oder verzweigte Alkylgruppe mit 1 bis 15, vorzugsweise 3 bis 10 C-Atomen, ist und R' die oben angegebene Bedeutung hat. Ferner bevorzugt sind Verbindungen entsprechend der Formel VId, worin eine oder beide die Ringe verknüpfenden Einfachbindungen durch eine Gruppe ausgewählt aus $-CH_2CH_2-$ -O-CO- oder -CO-O- ersetzt sind. Besonders bevorzugte Verbindungen der Formel VIc sind diejenigen der Formel VIc':

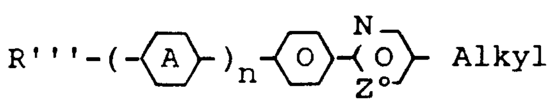

worin A 1,4-Phenylen oder trans-1,4-Cyclohexylen, Z° CH oder N, Alkyl eine geradkettige oder verzweigte Alkylgruppe mit 1 bis 15, vorzugsweise 3 bis 10 C-Atomen und n 0 oder 1 bedeutet.

Die Verbindungen der Formel I eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R'' II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G

-CH = CH- -N(0) = N-
-CH = CY- -CH = N(0)-
-C≡C- $-CH_2-CH_2-$
-C0-0- $-CH_2-0-$
-C0-S- $-CH_2-S-$
-CH = N- -C00-Phe-C00-
oder eine C-C-Einfachbindung,
Y Halogen; vorzugsweise Chlor, oder -CN, und

R' und R" Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $N0_2$, $CF_3$, F, Cl oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249-258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Fp. = Schmelzpunkt, Kp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

Herstellung von 5-Nonyl-2-p(5,5,6,6,7,7,8,8,9,9,10,10,10-tridecafluordecyloxyphenyl)-pyridin.

Zu 0,1 mol 5-Nonyl-2-p-hydroxyphenyl-pyridin gelöst in Methyl-ethylketon gibt man 0,3 mol Kaliumcarbonat und 0,11 mol 5,5,6,6,7,7,8,8,9,9,10,10,10-Tridecafluordecyl-1-tosylat und erhitzt das Reaktionsgemisch unter Rühren 12 Stunden am Rückfluß. Dann wird wie üblich aufgearbeitet.

Entsprechend wird 5-Octyl-2-p-hydroxyphenylpyrimidin verethert.

Beispiel 3 bis 86

Analog bzw. nach den angegebenen Syntheseschemata werden die folgenden Verbindungen hergestellt:

$(A^1-A^2 =$ 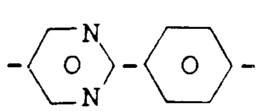 $R^1 = C_pH_{2p+1}-O-)$

| | p | Q | m | n | X |
|---|---|---|---|---|---|
| (3) | 6 | -O- | 3 | 4 | F |
| (4) | 7 | -O- | 3 | 4 | F |
| (5) | 8 | -O- | 3 | 4 | F |
| (6) | 9 | -O- | 3 | 4 | F |
| (7) | 10 | -O- | 3 | 4 | F |
| (8) | 11 | -O- | 3 | 4 | F |
| (9) | 12 | -O- | 3 | 4 | F |
| (10) | 6 | -O- | 3 | 5 | F |
| (11) | 7 | -O- | 3 | 5 | F |
| (12) | 8 | -O- | 3 | 5 | F |
| (13) | 9 | -O- | 3 | 5 | F |
| (14) | 10 | -O- | 3 | 5 | F |
| (15) | 11 | -O- | 3 | 5 | F |
| (16) | 12 | -O- | 3 | 5 | F |
| (17) | 6 | -O- | 3 | 6 | F |
| (18) | 7 | -O- | 3 | 6 | F |
| (19) | 8 | -O- | 3 | 6 | F |
| (20) | 9 | -O- | 3 | 6 | F |
| (21) | 10 | -O- | 3 | 6 | F |
| (22) | 11 | -O- | 3 | 6 | F |
| (23) | 12 | -O- | 3 | 6 | F |
| (24) | 6 | -O- | 4 | 5 | F |
| (25) | 7 | -O- | 4 | 5 | F |
| (26) | 8 | -O- | 4 | 5 | F |
| (27) | 9 | -O- | 4 | 5 | F |
| (28) | 10 | -O- | 4 | 5 | F |
| (29) | 11 | -O- | 4 | 5 | F |
| (30) | 12 | -O- | 4 | 5 | F |
| (31) | 6 | -O- | 3 | 5 | H |
| (32) | 7 | -O- | 3 | 5 | H |
| (33) | 8 | -O- | 3 | 5 | H |
| (34) | 9 | -O- | 3 | 5 | H |
| (35) | 10 | -O- | 3 | 5 | H |
| (36) | 11 | -O- | 3 | 5 | H |
| (37) | 12 | -O- | 3 | 5 | H |
| (38) | 6 | -O- | 5 | 6 | F |
| (39) | 7 | -O- | 5 | 6 | F |
| (40) | 8 | -O- | 5 | 6 | F |
| (41) | 9 | -O- | 5 | 6 | H |
| (42) | 10 | -O- | 5 | 6 | F |

| | p | Q | m | n | X |
|---|---|---|---|---|---|
| (43) | 11 | -O- | 5 | 6 | F |
| (44) | 12 | -O- | 5 | 6 | F |
| (45) | 6 | -O- | 5 | 5 | F |
| (46) | 7 | -O- | 5 | 5 | F |
| (47) | 8 | -O- | 5 | 5 | F |
| (48) | 9 | -O- | 5 | 5 | F |
| (49) | 10 | -O- | 5 | 5 | F |
| (50) | 11 | -O- | 5 | 5 | F |
| (51) | 12 | -O- | 5 | 5 | F |
| (52) | 6 | -O- | 1 | 4 | F |
| (53) | 7 | -O- | 1 | 4 | F |
| (54) | 8 | -O- | 1 | 4 | F |
| (55) | 9 | -O- | 1 | 4 | F |
| (56) | 10 | -O- | 1 | 4 | F |
| (57) | 11 | -O- | 1 | 4 | F |
| (58) | 12 | -O- | 1 | 4 | F |
| (59) | 6 | -O- | 1 | 5 | F |
| (60) | 7 | -O- | 1 | 5 | F |
| (61) | 8 | -O- | 1 | 5 | F |
| (62) | 9 | -O- | 1 | 5 | F |
| (63) | 10 | -O- | 1 | 5 | F |
| (64) | 11 | -O- | 1 | 5 | F |
| (65) | 12 | -O- | 1 | 5 | F |
| (66) | 6 | -O- | 1 | 6 | F |
| (67) | 7 | -O- | 1 | 6 | F |
| (68) | 8 | -O- | 1 | 6 | F |
| (69) | 9 | -O- | 1 | 6 | F |
| (70) | 10 | -O- | 1 | 6 | F |
| (71) | 11 | -O- | 1 | 6 | F |
| (72) | 12 | -O- | 1 | 6 | F |
| (73) | 6 | -O- | 2 | 5 | F |
| (74) | 7 | -O- | 2 | 5 | F |
| (75) | 8 | -O- | 2 | 5 | F |
| (76) | 9 | -O- | 2 | 5 | F |
| (77) | 10 | -O- | 2 | 5 | F |
| (78) | 11 | -O- | 2 | 5 | F |
| (79) | 12 | -O- | 2 | 5 | F |
| (80) | 6 | -O- | 1 | 5 | H |
| (81) | 7 | -O- | 1 | 5 | H |
| (82) | 8 | -O- | 1 | 5 | H |
| (83) | 9 | -O- | 1 | 5 | H |
| (84) | 10 | -O- | 1 | 5 | H |
| (85) | 11 | -O- | 1 | 5 | H |
| (86) | 12 | -O- | 1 | 5 | H |

## Patentansprüche

**1.** Chirale oder achirale Ringverbindungen der Formel I

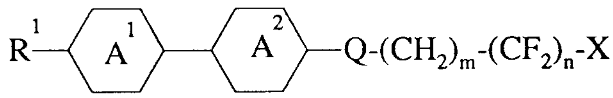

I

worin

R[1] eine geradkettige Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH = CH-Gruppen und/oder-CHHalogen- und/oder -CHCN-Gruppen und/oder -O-CO-CHHalogen- und/oder -CO-O-CHCN-Gruppen ersetzt sein können, oder $X-(CF_2)_n-(CH_2)_m-Q-$,

X H oder F,

A[1] und A[2] jeweils unabhängig voneinander unsubstituiertes oder durch ein oder zwei F-Atome substituiertes 1,4-Phenylen, worin auch eine oder zwei CH-Gruppen durch N ersetzt sein können,

Q -O-,

m 1 bis 10, und

n 2 bis 8 bedeutet, mit der Maßgabe, daß die Verbindung der Formel

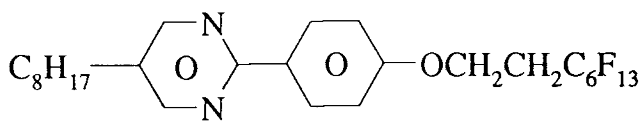

ausgenommen ist.

**2.** Ringverbindungen nach Anspruch 1, worin

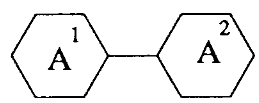

,

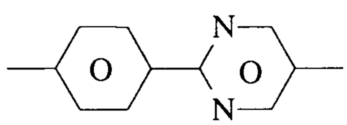

oder

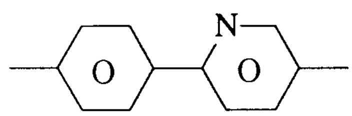

bedeuten,

wobei die 1,4-Phenhylengruppen durch ein oder zwei Fluoratome substituiert sein können.

**3.** Chirale getiltete smektische flüssigkristalline Phase mit mindestens zwei flüssigkristallinen Komponenten, dadurch gekennzeichnet, daß sie mindestens eine Verbindung der Formel I nach Anspruch 1 oder 2 enthält.

**4.** Chirale getiltete smektische flüssigkristalline Phase mit mindestens zwei flüssigkristallinen Komponenten, dadurch gekennzeichnet, daß sie mindestens eine Verbindung der Formel I

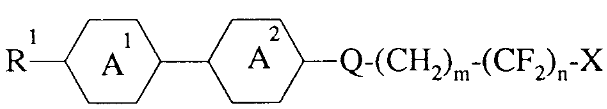

I

worin

| | |
|---|---|
| $R^1$ | eine geradkettige Alkyl- oder Perfluoralkyl-Gruppe mit jeweils 1-12 C-Atomen, worin auch eine oder zwei nicht benachbarte $CH_2$- bzw. $CF_2$-Gruppen durch O-Atome und/oder -CO-Gruppen und/oder -CO-O-Gruppen und/oder -CH = CH-Gruppen und/oder-CHHalogen- und/oder -CHCN-Gruppen und/oder -O-CO-CHHalogen- und/oder -CO-O-CHCN-Gruppen ersetzt sein können, oder $X-(CF_2)_n-(CH_2)_m-Q-$, |
| X | H oder F, |
| $A^1$ und $A^2$ | jeweils unabhängig voneinander unsubstituiertes oder durch ein oder zwei F-Atome substituiertes 1,4-Phenylen, worin auch eine oder zwei CH-Gruppen durch N ersetzt sein können, |
| Q | -O-, |
| m | 1 bis 10, und |
| n | 2 bis 8 bedeutet, |

enthält, mit der Maßgabe, daß Phasen mit 0,01 bis 1 Gew.-% einer Verbindung der Formel I ausgenommen sind.

**5.** Chirale getiltete smektische flüssigkristalline Phase nach Anspruch 4, dadurch gekennzeichnet, daß sie 10 bis 95 % einer oder mehrerer Verbindungen der Formel I enthalten.

**6.** Verwendung der Verbindungen der Formel I nach Anspruch 1 oder 2 als Komponenten flüssigkristalliner Phasen.

**7.** Elektrooptisches Anzeigeelement, dadurch gekennzeichnet, daß es als Dielektrikum eine Phase nach einem der Ansprüche 3 bis 5 enthält.

## Claims

**1.** Chiral or achiral ring compounds of the formula I

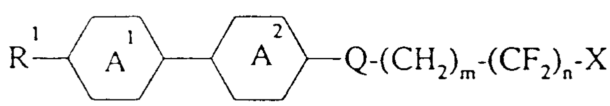

I

in which

| | |
|---|---|
| $R^1$ | is a straight-chain alkyl or perfluoroalkyl group, in each case having 1-12 C atoms, in which, in addition, one or two non-adjacent $CH_2$ or $CF_2$ groups may be replaced by O atoms and/or -CO- groups and/or -CO-O- groups and/or -CH = CH-groups and/or -CHhalogen- and/or -CHCN- groups and/or -O-CO-CHhalogen and/or -CO-O-CHCN-groups, or is $X-(CF_2)_n-(CH_2)_m-Q-$, |
| X | is H or F |
| $A^1$ and $A^2$ | , in each case independently of one another, are 1,4-phenylene which is unsubstituted or substituted by one or two F atoms, in which, in addition, one or two CH groups may be replaced by N, |
| Q | is -O-, |
| m | is 1 to 10, and |
| n | is 2 to 8, |

with the proviso that the compound of the formula

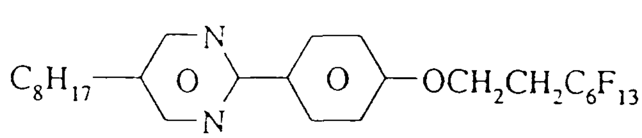

is excluded.

2. Ring compounds according to Claim 1, in which

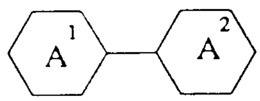 , is 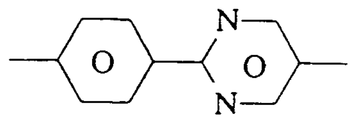

or 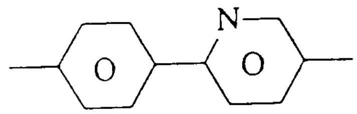 ,

where the 1,4-phenhylene [sic] groups can be substituted by one or two fluorine atoms.

3. Chiral tilted smectic liquid-crystalline phase having at least two liquid-crystalline components, characterized in that it contains at least one compound of the formula I according to Claim 1 or 2.

4. Chiral tilted smectic liquid-crystalline phase containing at least two liquid-crystalline components, characterized in that it contains at least one compound of the formula I

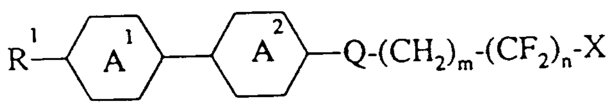 I

in which

| | |
|---|---|
| $R^1$ | is a straight-chain alkyl or perfluoroalkyl group, in each case having 1-12 C atoms, in which, in addition, one or two non-adjacent $CH_2$ or $CF_2$ groups may be replaced by O atoms and/or -CO- groups and/or -CO-O- groups and/or -CH=CH-groups and/or -CHhalogen- and/or -CHCN- groups and/or -O-CO-CHhalogen and/or -CO-O-CHCN-groups, or is $X-(CF_2)_n-(CH_2)_m-Q-$, |
| X | is H or F |
| $A^1$ and $A^2$ | , in each case independently of one another, are 1,4-phenylene which is unsubstituted or substituted by one or two F atoms, in which, in addition, one or two CH groups may be replaced by N, |
| Q | is -O-, |
| m | is 1 to 10, and |
| n | is 2 to 8, |

with the proviso that phases containing from 0.01 to 1% by weight of a compound of the formula I are excluded.

5. Chiral tilted smectic liquid-crystalline phase according to Claim 4, characterized in that it contain [sic] from 10 to 95% of one or more compounds of the formula I.

6. Use of the compounds of the formula I according to Claim 1 or 2 as components of liquid-crystalline phases.

7. Electrooptical display element, characterized in that it contains, as dielectric, a phase according to one of Claims 3 to 5.

## Revendications

**1.** Composés cycliques chiraux ou achiraux de formule I :

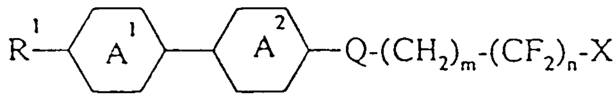

I

dans laquelle :

- $R^1$ représente un groupe alkyle ou perfluoroalkyle à chaîne linéaire ayant chacun 1-12 atomes de carbone, dans lequel aussi 1 ou 2 groupes non-voisins $CH_2$ ou $CF_2$ peuvent être remplacés par des atomes de O et/ou des groupes CO et/ou des groupes -CO-O- et/ou des groupes -CH=CH et/ou des groupes -CHHalogène-et/ou -CHCN- et/ou des groupes -O-CO-CHHalogène et/ou -CO-O-CHCN-, ou $X-(CF_2)_n-(CH_2)_m-Q-$,
- X représente H ou F,
- $A^1$ et $A^2$ représentent chacun indépendamment l'un de l'autre un groupe 1,4-phénylène non substitué ou substitué par un ou deux atomes de F, dans lequel aussi un ou deux groupes CH peuvent être remplacés par N,
- Q représente -O-,
- m représente 1 à 10, et
- n représente 2 à 8

à la condition qu'on exclue le composé de formule

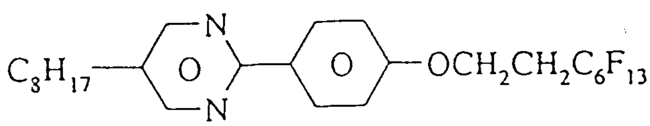

**2.** Composés cycliques selon la revendication 1, dans lesquels

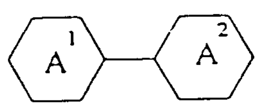

représente

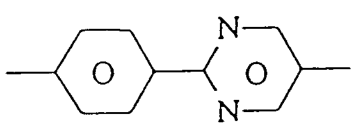

ou

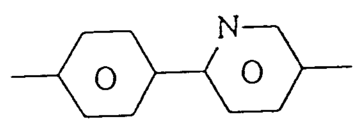

dans lesquels les groupes 1,4-phénylène peuvent être substitués par 1 ou 2 atomes de fluor.

**3.** Phase de cristaux liquides smectique inclinée chirale avec au moins deux composants de cristaux liquides, caractérisée en ce qu'elle contient au moins un composé de formule I selon la revendication 1 ou 2.

**4.** Phase de cristaux liquides smectique inclinée chirale avec au moins deux composants de cristaux liquides, caractérisée en ce qu'elle contient au moins un composé de formule I

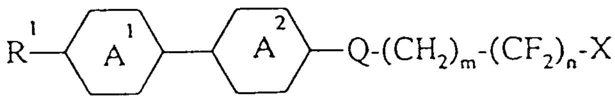

I

dans laquelle

- $R^1$ représente un groupe alkyle ou perfluoroalkyle à chaîne linéaire ayant chacun 1-12 atomes de carbone, dans lequel aussi 1 ou 2 groupes non-voisins $CH_2$ ou $CF_2$ peuvent être remplacés par des atomes de O et/ou des groupes CO et/ou des groupes -CO-O- et/ou des groupes -CH=CH et/ou des groupes -CHHalogène-et/ou -CHCN- et/ou des groupes -O-CO-CHHalogène et/ou -CO-O-CHCN-, ou $X\text{-}(CF_2)_n\text{-}(CH_2)_m\text{-}Q\text{-}$,
- X représente H ou F,
- $A^1$ et $A^2$ représentent chacun indépendamment l'un de l'autre un groupe 1,4-phénylène non substitué ou substitué par un ou deux atomes de F, dans lequel aussi un ou deux groupes CH peuvent être remplacés par N,
- Q représente -O-,
- m représente 1 à 10, et
- n représente 2 à 8,

à la condition que les phases avec 0,01 à 1 % en poids d'un composé de formule I sont exclues.

**5.** Phase de cristaux liquides smectique inclinée chirale selon la revendication 4, caractérisée en ce qu'elle contient 10 à 95% d'un ou plusieurs composés de formule I.

**6.** Utilisation des composés de formule I selon la revendication 1 ou 2 comme composants de phases de cristaux liquides.

**7.** Elément d'affichage électro-optique, caractérisé en ce qu'il contient comme diélectrique une phase selon l'une des revendications 3 à 5.